(19) Europäisches Patentamt
European Patent Office
Office européen des brevets

(11) Publication number: **0 019 067**
**B1**

(12) **EUROPEAN PATENT SPECIFICATION**

(45) Date of publication of patent specification: **06.10.82**

(21) Application number: **80101662.7**

(22) Date of filing: **27.03.80**

(51) Int. Cl.³: **C 07 D 487/14,**
C 07 D 499/68,
C 07 D 501/20
//(C07D487/14, 241/00,
235/00, 235/00)

(54) **5,10-Dioxo-5,10-dihydrodiimidazo(1,5-a,1',5'-d)pyrazine-1,6-dicarboxylic acid and a process for producing imidazoledicarboxylic acid amido-derivatives.**

(30) Priority: **27.03.79 JP 36133/79**

(43) Date of publication of application:
**26.11.80 Bulletin 80/24**

(45) Publication of the grant of the patent:
**06.10.82 Bulletin 82/40**

(84) Designated Contracting States:
**CH DE GB IT**

(56) References cited:
**DE - A - 2 826 546**

**Chemical Abstracts vol. 84, no. 15, 12 April 1976 Columbus, Ohio, USA K. TAKAHASHI et al. "Cyclic dimerization of imidazole carboxylic acids and cleavage of the dimer" page 569, column 1, abstract no. 105479a**

**Patents Abstracts of Japan vol. 3, no. 50, 27 April 1979 page 160 C 44**

(73) Proprietor: **AJINOMOTO CO., INC.**
**5-8, Kyobashi 1-chome, Chuo-ku**
**Chiyoda-ku Tokyo 104 (JP)**

(72) Inventor: **Yasuda, Naohiko**
**2-36-2, Hairando**
**Yokosuka-shi Kanagawa-ken (JP)**
Inventor: **Nakanishi, Eiji**
**2-20-8, Kannon, Kawasaki-ku**
**Kawasaki-shi Kanagawa-ken (JP)**

(74) Representative: **Schübel-Hopf, Ursula et al,**
**Patentanwälte Schiff, von Füner, Strehl, Schübel-Hopf Ebbinghaus, Finck Mariahilfplatz 2 u. 3**
**D-8000 München 90 (DE)**

**EP 0 019 067 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

5,10-Dioxo-5,10-dihydrodiimidazo[1,5-a,1′,5′-d]pyrazine-1,6-dicarboxylic acid and a process for producing imidazoledicarboxylic acid amido-derivatives

The invention relates to a novel reactive imidazole dicarboxylic acid derivative, which can be used as a convenient reactant for forming derivatives of $\alpha,\beta$-imidazoledicarboxylic acid.

It is known that imidazoledicarboxylic acid derivatives of amino group-containing biologically active substances have a superior antibacterial activity compared with the unmodified amino group-containing biologically active substance.

For example a compound of the general formula

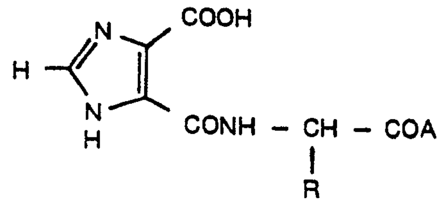

which is hereinafter referred to as "compound modified with imidazoledicarboxylic acid," has a much superior action concerning the antibacterial activity, particularly antibacterial activity against Pseudomonas aeruginosa in comparison with a compound which has not been modified with the imidazoledicarboxylic acid, having the following general formula:

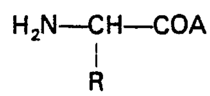

In the above general formulae R is a hydrogen atom, an alkyl group, an aralkyl group, an aryl group or a heteroaromatic group, which may have substituent group or groups.

Preferably R is a group of the formula

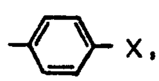

in which X is a hydrogen atom or a hydroxyl group.

Amino acids from which the compounds having the above formula can be derived are, for example, phenylglycine, (4-hydroxyphenyl) glycine etc., and are in the L-, D- and DL-form. In many cases, D-form is more suitable in view of antibacterial activity.

A is an organic residue having the following formula:

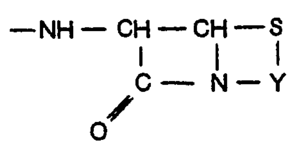

wherein Y is a divalent organic residue having one of the following formulae:

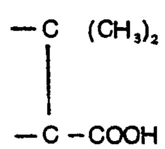

and

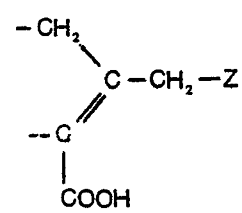

and wherein the bivalent residue Y is bound in the residue A in such a manner that the carbon atom which carries the carboxyl group is bound to the nitrogen atom, and wherein Z is a hydrogen atom, an acyloxy group such as an acetoxy group, 9 carbamoyloxy group, a heteroaromatic thio group such as the 5-(1-methyltetrazolyl)-thio-group or a quaternary ammonium group such as a substituted or non-substituted pyridinium group, a quinolinum group or a picolinium group.

The above compounds can be produced by modifying $\alpha$-aminopenicillins or $\alpha$-aminocephalosporins having the following general formula:

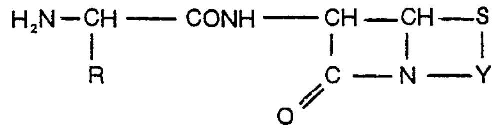

with $\alpha$,$\beta$-imidazoledicarboxylic acid by a condensation reaction with a reactive derivative of imidazoledicarboxylic acid having the following formula:

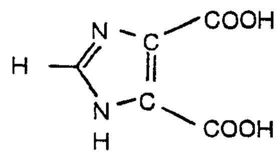

However, when condensation reactions are carried out using a known reactive derivative such as the acid chloride derivative thereof as a modifying agent, more than 2 moles of the acid chloride derivative per 1 mole of starting material is needed, many side reactions arise, and the yield is low.

It is therefore object of this invention to provide a convenient and effective reactive derivative of imidazoledicarboxylic acid, which is a particularly good reactant for modifying amino group-containing compounds, specifically the above-mentioned $\alpha$-amino-penicillins and cephalosporins.

A further object of the invention is the use of this reactive derivative of imidazoledicarboxylic acid to produce amino group-containing substances modified with imidazoledicarboxylic acid, specifically for producing imidazoledicarboxylic acid derivatives of the above-stated $\alpha$-aminopenicillins or cephalosporins.

From Nippon Kagaku Kaishi 1975 (12), 2244—5 (Japan) and the corresponding reference in "Chemical Abstracts" Vol. 1976 ref. 84: 105479a, it was already known that imidazole-4-carboxylic acid and imidazole-4,5-dicarboxylic acid may be reacted with $PCl_5$ or $SOCl_2$ to give diimidazo[1,5-a,1',5'-d]pyrazine-4,9-dione and 1,9-dioxo-4,9-dihydrodiimidazo [1,5-a, 1',5'-d]pyrazine-1,6-dicarbonyldichloride.

The latter compounds may be reacted with amines to form the corresponding amides of imidazole-4-carboxylic acid and imidazole-4,5-dicarboxylic acid. The present inventors have found that the condensation product of imidazole-4,5-dicarboxylic acid, which is a new compound, may be favourably used for producing imidazole dicarboxylic acid derivatives of $\alpha$-aminopenicillin and cephalosporins.

This invention relates to a novel compound, 5,10-dioxo-5,10-dihydrodiimidazo[1,5-a, 1'5'-d] pyrazine-1,6-dicarboxylic acid having the following formula:

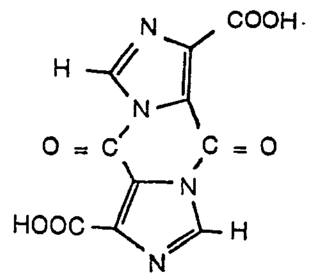

(I)

which may be used as a good reactive derivative of $\alpha$,$\beta$-imidazoledicarboxylic acid.

Further the invention relates to a process for producing an imidazoledicarboxylic acid derivative having the following formula

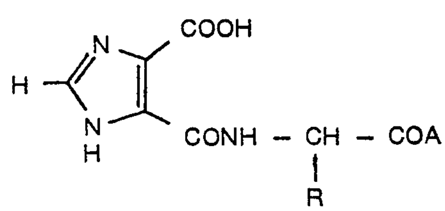

(II)

wherein R and A are as defined above, which process comprises reacting the above-defined imidazoledicarboxylic acid derivative of the formula I with a compound having the following formula

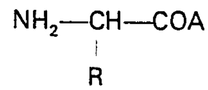

wherein A and R are as defined above.

According to a preferred embodiment R is a group

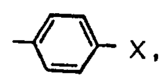

in which X is a hydrogen atom or a hydroxyl group.

In the process carboxylic groups which are in the form of ester groups may be optionally converted to the free carboxylic acid groups and/or converted to the corresponding salts with pharmaceutically acceptable salt-forming bases, such as non-toxic salts of alkali metals, alkaline earth metals, ammonium salts and salts or organic amines.

The imidazoledicarboxylic acid derivative of the present invention is prepared, for example, by hydrolyzing the corresponding acid halid having the following formula:

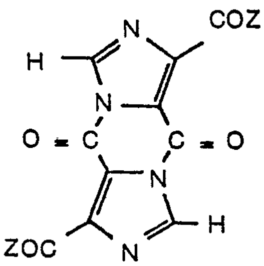

(III)

wherein Z is a halogen atom, preferably a chlorine atom. Such compound is hydrolyzed, for example, by reaction with water. This hydrolysis reaction is finished by stirring reaction mixture for about a few hours or one day under cooling or at room temperature and the object product can be obtained by removing insoluble material by filtration.

The hydrolysis of the acid halide of the formula III generally may be carried out by reacting the acid halide with water preferably at a temperature of 5 to 50°C for 5 to 24 hours.

The acid halide of the formula III may be produced from $\alpha,\beta$-imidazoledicarboxylic acid by reacting the dicarboxylic acid with a halogenating agent for the hydroxyl group of the carboxyl group such as thionyl chloride. phosphorus oxychloride and phosphorus pentachloride, in the presence of an inactive organic solvent such as benzene, hexane, ethylene dichloride and dioxane. This reaction preferably is carried out at a temperature of 50°C up to the boiling point of the solvent used.

The compound of the present invention reacts with amines ($H_2N—R'$) very well and therefore is extremely useful as modifying agent for producing imidazoledicarboxylic acid monoamides.

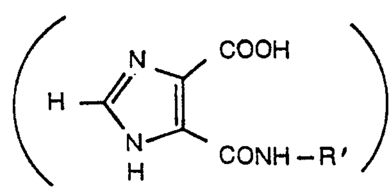

particularly, for producing the above materials modified with imidazoledicarboxylic acid. For example, the yield of a known process using the acid chloride (JA—OS 5974/1979 and DE—OS 2826546) is from 10 to 40%. On the contrary, that of the process of this invention is from 45 to 75%.

In the reaction of the imidazoledicarboxylic acid derivative of the present invention with the above $\alpha$-aminopenicillins or cephalosporins, the $\alpha$-aminopenicillins or cephalosporins are dissolved in a solvent in the presence of base such as an kalimetal bicarbonate, alkali metal carbonate, trialkyl amine or pyridine and then the compound of the present invention is added thereto. Examples of the solvents employed for reaction are water, acetone, dioxane, acetonitrile, chloroform, dichloromethane, tetrahydrofuran (THF), and dimethylformamide (DMF). Hydrophilic organic solvents can be used in mixture with water. The reaction temperature may be generally a temperature of 0°C to 40°C. 2 moles of $\alpha$-aminopenicillins or cephalosporins are preferably used per one mole of the compound of the present invention.

In the above reaction, the above $\alpha$-aminopenicillins or cephalosporins wherein carboxyl groups in the group Y are changed to the ester form, for example, a tert. butyl ester, benzyl ester, silyl ester,

trichloroethyl ester or diphenylmethyl ester, can be used for reaction. These ester groups of esters, obtained by the reaction can be removed by conventional methods to give free carboxyl groups.

The materials modified with imidazoledicarboxylic acid wherein A is the residue of cephalosporin and further Z is a heteroaromatic thio group or tert.-ammonium group, can be produced by synthesizing cephalosporins wherein Z is an acetoxy group and then substituting the acetoxy group by a heteroaromatic thio group or quaternary ammonium group.

Reaction products can be isolated by using an isolating means which is known as such, extraction, column chromatography or recrystallization.

An example for producing the imidazoledicarboxylic acid derivative of the present invention is shown as follows:

Example 1

Imidazoledicarboxylic acid (7.8 g, 0.05 M) was suspended in dry benzene (100 ml), and thereto DMF (4 ml) and then thionyl chloride (30 ml) were added. The thus obtained mixture was refluxed at a temperature of 85°C while stirring. The final reaction mixture was concentrated to yield a solid material. The obtained material was added to dry benzene (50 ml) and the mixture was again concentrated to yield a solid material. To the remaining material, benzene (50 ml) was added and the mixture was stirred at room temperature for 30 minutes. An insoluble solid material was recovered by filtration and then washed with benzene and dried under reduced pressure to give the object product, 5,10-dioxo-5,10-dihydrodiimidazo[1,5-a, 1'5'-d] pyrazine-1,6-dicarboxylic acid chloride (7.0 g, yield· 89%).

Elemental analysis:
Found C 38.03%, H 0.74%, N17.81%, Cl 22.37%
Calcd. as $C_{10}H_2N_4O_4Cl_2$
C 38.36%, H 0.64%, N 17.90%, Cl 22.65%

The obtained acid chloride (7.0 g) was suspended in water (150 ml) and the mixture was stirred at 10 to 20°C overnight. An insoluble solid material was obtained by filtration and washed with water, a small amount of THF and then ether. The material was dried under reduced pressure to give the object product, 5,10-dioxo-5,10-dihydrodiimidazo[1,5-a, 1'5'-d] pyrazine-1,6-dicarboxylic acid. 2 hydrate (7.0 g, yield: 100%).

M.P., 248°C (dec.)

I.R. spectrum:
3500 $cm^{-1}$, 1750 $cm^{-1}$, 1710 $cm^{-1}$, 1255 $cm^{-1}$, 930 $cm^{-1}$

Elemental analysis:
Found C 38.65%, H 2.40%, N 18.02%,
Calcd. as $C_{10}H_4N_4O_6 \cdot 2H_2O$
C 38.47%, H 2.58%, N 17.95%

An example for reacting an amine with the compound of the present invention produced above, is shown as follows:

Example 2

Benzylamine (1.2 g, 11 mM) was dissolved in dichloromethane (50 ml) and triethylamine (2.8 ml, 20 mM) was added thereto. To this mixture 5,10-dioxo-5,10-dihydrodiimidazo [1,5-a, 1',5'-d] pyrazine-1,6-dicarboxylic acid. 2 hydrate (1.1 g, 3.6 mM) was added and the mixture was stirred at 40°C for 5 hours. To the reaction mixture ether (100 ml) was added and the precipitated crystals were obtained by filtration. The thus obtained crystals were added to water (100 ml) and dissolved by adjusting the water solution to PH7 by adding 2N—NaOH. The obtained solution was washed with ethyl acetate. The water phase was adjusted to pH2 with 2N—HCl and the precipitated crystalline substance was obtained by filtration and dried. The crystalline substance was dissolved in THF (250 ml) at 50°C, and an insoluble material was separated by filtration. The obtained mother liquid was concentrated and ether-petroleum ether was added thereto to precipitate a crystalline material. After cooling overnight, the precipitated crystals were obtained by filtration and washed with petroleum ether. The crystals were dried under reduced pressure to give the object product, imidazole-4-N-benzylcarboxamide-5-carboxylic acid (1.2 g, yield: 68%).

Elemental analysis:
Found C 58.80%, H 4.51%, N 17.16%
Calcd. as $C_{12}H_{11}N_3O_3$
C 58.77%, H 4.52%, N 17.14%

'H—N.M.R. spectrum (solvent: DMSO—$D_2O$):

δ 4.58 (S. 2H) (—NH—$CH_2$— 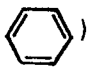)

7.20 ∼ 7.45 (m. 5H) (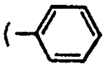)

8.05 (S. 1H) (—H in the second position of imidazole group)

The following experiments show that the imidazoledicarboxylic acid derivative of the present invention is a very good reactive derivative of imidazoledicarboxylic acid:

Examples 3 to 6

The compounds to be modified, which are shown in table 1 (16 mM) were suspended in dry dichloromethane (100 ml) and triethylamine (6 ml) was added thereto. The mixture was stirred for 30 minutes under cooling with ice-water. To this solution the compound of the present invention produced above, i.e. 5,10-dioxo-5,10-dihydrodiimidazo [1,5-a, 1',5'-d] pyrazine-1,6-dicarboxylic acid. 2 hydrate (2.2 g, 7 mM) was added while stirring and cooling. The mixture was stirred overnight and concentrated to give a solid material. This material was added to water (50 ml) and stirred to give a homogeneous solution. The solution was adjusted to pH 8 and 6% HCl aqueous solution, stirred for 10 minutes, and then washed with ethyl acetate (50 ml). The water phase was adjusted to pH 2 with 6% HCl aqueous solution, and the mixture was stirred fro 20 minutes. The precipitated crystals were obtained by filtration, washed with water, and then dried under reduced pressure at 40°C. The thus obtained solid material was suspended in a solvent (500 ml) obtained by mixing ethyl acetate and methanol (volume ratio: 1/1) and the mixture stirred at 40°C for 20 minutes. An insoluble material was separated by filtration. The thus obtained organic phase was concentrated under reduced pressure to a volume of 50 ml, and ether (500 ml) was added thereto. This mixture was kept overnight in the refrigerator. The precipitated crystals were recovered washed with petroleum ether and dried to give the object product.

Comparative Examples 1 to 4

In order to compare the process according to this invention with the known process the above experiment was repeated with the exception, that 5,10-dioxo-5,10-dihydrodiimidazo [1,5-a, 1',5'-d] pyrazine-1,6-dicarboxylic acid. 2 hydrate was replaced by 5,10-dioxo-5,10-dihydrodiimidazo [1,5-a, 1',5'-d] pyrazine-1,6-dicarboxylic acid chloride, which thereafter is called the dichloride derivative. The obtained results are listed in Table 1.

The reaction was carried out as follows:

The material to be modified (100 mM) was suspended in dry dichloromethane (40 ml) and triethylamine (14 ml) was added thereto. The mixture was stirred for 30 minutes under cooling. On the other hand, the dichloride derivative (6.3 g, 10 mM) was suspended in dry dichloromethane (40 ml) and to this mixture a dichloromethane solution of a material to be modified, which had been previously produced, was added dropwise slowly over 15 minutes. After completing the reaction, the mixture was further stirred for 2 hours under cooling with ice-water. Insoluble material was removed by filtration from the reaction solution and the obtained solution was concentrated under reduced pressure at 30°C or less to yield a solid material. The thus obtained material was dissolved in water (70 ml) and then ethyl acetate (70 ml) was added thereto to form two phases. The ethyl acetate phase was removed and to the obtained aqueous phase ethyl acetate (100 ml) and thereto 6% HCl aqueous solution were added while stirring to adjust the aqueous phase to pH 2. The precipitated insoluble material was removed by filtration and a solution having two phases was obtained. From the aqueous phase, organic material was extracted with ethyl acetate (100 ml) once more. All obtained ethyl acetate phases were combined and dried over magnesium sulfate. The ethyl acetate solution was concentrated at 30°C or less and to the thus obtained solid material ether (100 ml) was added to precipitate a powder. The powder was obtained by filtration and dried to give the object product.

TABLE 1

| Example | Material to be modified (Starting Material) | Object Product | Yield | |
|---|---|---|---|---|
| | | | Compound of Present Invention | Dichloride Derivative |
| Example 3 and comparative example 1 | Anhydrous 7$\beta$-[D(—)-$\alpha$-aminophenylacetamido] cephalosporanic acid | 7$\beta$-[D(—)-$\alpha$-(4-carboxyimidazole-5-carboxamido)phenyl-acetoamide]cephalosporanic acid | 75% | 34% |
| Example 4 and comparative example 2 | Anhydrous D(—)-$\alpha$-aminobenzyl-penicillin | D-$\alpha$-(4-carboxy-imidazole-5-carboxamido) benzyl-penicillin | 74% | 12% |
| Example 5 and comparative example 3 | D(—)-$\alpha$-amino-p-hydroxybenzyl-penicillin.3$H_2O$ | D-$\alpha$-(4-carboxy-imidazole-5-carboxamido)-p-hydroxy-benzyl-penicillin. 1$H_2O$ | 45% | 8% |
| Example 6 and comparative example 4 | Anhydrous 7$\beta$-7[D (—)-$\alpha$-amino-p-hydroxyphenyl-acetamido] cephalosporanic acid | 7$\beta$-[D(—)-$\alpha$-(4-carboxyimidazole-5-carboxamido)-p-hydroxyphenylacet-amide] cephalos poranic acid | 72% | 10% |

In the following the physical properties of the products obtained in the above examples 3 to 6 are described:

Product of Example 3

Elemental analysis:
Found C 49.66%, H 3.98%, N 12.77%, S 5.66%
Calcd. for $C_{23}H_{21}N_5O_9S \cdot 1H_2O$
C 49.19%, H 4.14%, N 12.47%, S 5.71%

TLC (silica gel) $R_f = 0.70$
I.R. spectrum (Nujol)

$\nu_{CO}(\beta\text{-lactam}) = 1775\ cm^{-1}$

$\nu_{CO}(-OCOCH_3) = 1745\ cm^{-1}$

N.M.R. spectrum of the disodium salt (Solvent; D20)

ppm 2.13 (s, 3H) (—$OCOCH_3$)

3.36 (m, 2H) (>$CH_2$, 2nd position)

5.02 (d, 1H) (—H, 6th position)

5.73 (m, 2H) (—H, 7th position) —CH—)

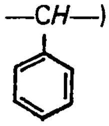

7.50 (bs, 5H) ( 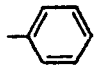 )

7.80 (s, H) (—H, 2nd position of imidazole)

Product of Example 4

Elemental analysis:
Found C 50.77%, H 4.51%, N 14.00%, S 6.35%
Calcd. for $C_{21}H_{21}N_5O_7S$
C 51.73%, H 4.35%, N 14.37%, S 6.58%

Thin Layer Chromatography (TLC) (silica gel) $R_f = 0.45$
I.R. spectrum (Nujol)
$\nu_{CO}$($\beta$-lactam) = 1773 $cm^{-1}$

The thus obtained compound was converted to the disodium salt thereof by the following method.

D-$\alpha$-(4-carboxyimidazole-5-carboxamido)benzylpenicillin (0.5 g, 1.0 mM) was dissolved in a mixture of methanol (15 ml) and ethyl acetate (10 ml) and to the solution thus obtained 2-ethylhexane carboxylic acid sodium salt n-butanol solution (2M/1) (1.23 ml) was added. The mixture was stirred for 10 minutes. Crystallized material was produced by adding ethyl acetate (150 ml) dropwise to the solution. Crystals were obtained by filtration and dried, and thereby D - $\alpha$ - (4 - carboxyimidazole - 5 - carboxamido)benzylpenicillin disodium salt monohydrate [I] (0.35 g) was obtained.

Elemental analysis:
Found C 46.67%, H 4.19%, N 12.32%
Calcd. for $C_{21}H_{19}N_5O_7SNa_2 \cdot 1H_2O$
C 45.90%, H 3.86%, N 12.74%

N.M.R. spectrum ($D_2O$, $\delta$)

ppm 1.50 (b.s. 6H) ( 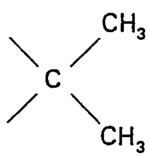 , 2nd position)

4.30 (s, 1H) (—H, 3rd position)

5.40—5.80 (m. 3H) (—*CH*, —H 5th and 6th position)

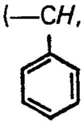

7.47 (b.s. 5H) ( 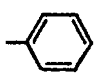 )

7.82 (s, 1H) (—H, 2nd position in imidazole)

Product of Example 5

Elemental analysis:
Found C 48.82%, H 4.50%, N 13.03%
Calcd. as $C_{21}H_{21}N_5SO_8 \cdot 1H_2O$
C 48.35%, H 4.45%, N 13.43%

I.R. spectrum (Nujol):
$\nu_{C=O}$ ($\beta$-lactam) = 1770 $cm^{-1}$
N.M.R. spectrum (solvent: DMSO—$D_2O$):

1.42 (s. 3H)
1.56 (s. 3H)
} 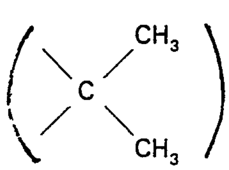

4.23 (s. 1H) (—H in the third position)

5.37 (d. 1H) (—H in the 5th position)

5.50 (d. 1H) (—H in the 6th position)

5.70 (s. 1H) 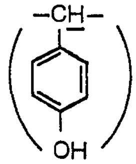

6.75 (d. 2H)
7.30 (d. 2H) 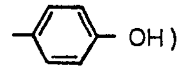

8.06 (s. 1H) (—H in the second position of imidazole group)

Product of Example 6

I.R. spectrum (Nujol):
$\nu_{c=o}$ ($\beta$-lactam) = 1775 $cm^{-1}$
$\nu_{c=o}$ ($-OCOCH_3$) = 1730 $cm^{-1}$
N.M.R. spectrum (Solvent: DMSO—$D_2O$)

$\delta$ 2.03 (s. 3H) ($-OCOCH_3$)

3.46 (m. 2H) (= $CH_2$ in the second position)

4.82 (q. 2H) (= $CH_2$ in the third position)

5.03 (d. 1H) (—H in the 6th position)

5.70 (s. 1H) 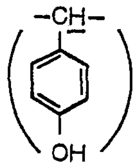

5.76 (d. 1H) (—H in the 7th position)

6.80 (d. 2H)
7.36 (d. 2H) 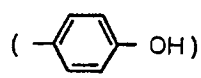

8.06 (s. 1H) (—H in the second position of imidazole group)

Example 7

7-$\beta$-[D-(—)-$\alpha$-(4-carboxyimidazole-5-carboxamido)phenylacetamido]-cephalosporanic acid.2Na salt (770 mg, 1.3 mM) as obtained in example 3 and converted to 2Na salt in the same manner as the object compound of example 4 and 1-methyl-5-mercapto-1H-tetrazole (150 mg, 1.3 mM) were dissolved in a phosphate buffer solution (10 ml) of pH 6.4. At this time, the solution was adjusted to pH 6.4 with 2N NaOH solution. This solution was reacted at 60°C with stirring for 24 hours. 5 hours after starting the reaction, the mixture was adjusted to pH 6.4 with 2N NaOH solution.

To the reaction mixture water (20 ml) was added and the solution was adjusted to pH 7. The aqueous solution was washed with ethyl acetate (30 ml). To the obtained aqueous layer ethyl acetate (50 ml) was added and 6% hydrochloric acid solution was added to adjust the aqueous layer to pH 2. An insoluble material was removed by filtration to yield a solution having 2 layers. The aqueous solution was extracted with ethyl acetate (50 ml), once more. The obtained ethyl acetate solutions were combined, washed with water and dried with anhydrous magnesium sulfate. The ethyl acetate solution was concentrated at less than 30°C and the residue was triturated with ether (50 ml). The triturated material was isolated by filtration and dried to give the desired compound. 7 - $\beta$ - [D - (—) - $\alpha$ - (4 - carboxyimidazole - 5 - carboxamido)phenylacetamido] - 3 - [[(1 - methyl - 1H - tetrazol - 5 - yl)thio]methyl] - 3 - cephem - 4 - carboxylic acid monohydrate (160 mg, yield: 27%).

The 2 Na salt of this compound was produced in the same manner as the compound of example 4.

Elemental analysis:
Found C 43.01%, H 3.56%, Na 6.33%
Calcd. for $C_{23}H_{19}N_9O_7S_2Na_2$
C 41.81%, H 3.06%, Na 6.96%
TLC (silica gel) $R_f = 0.23$
I.R. spectrum (Nujol):
$\nu_{C=O}$($\beta$-lactam) = 1765 $cm^{-1}$
N.M.R. spectrum ($D_2O$, $\delta$)

ppm 3.10—3.60 (m, 2H) (>$CH_2$, 2nd position)
3.88 (s, 3H) (—$CH_3$ in the tetrazole)
4.00—4.20 (m, 2H) (—$CH_2$—S—, 3rd position)
4.93 (d, 1H) (—H, 6th position)
5.31—5.67 (m, 2H) (—H, 7th position; –CH–)
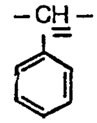
7.30 (bs, 5H) ( 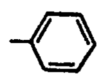 )
7.63 (s, 1H) (—H, 2nd position in imidazole)

## Example 8

7-$\beta$-[D-(—)-$\alpha$-(4-carboxyimidazole-5-carboxamido)phenylacetamido]-cephalosporanic acid.2Na salt (1.17 g, 2 mM) was dissolved in water (12 ml) and 4-pyridine-ethanesulfonic acid (0.75 g, 4 mM) was added thereto. The solution was adjusted to pH 7 with 2N NaOH solution. To this solution sodium iodide (8.3 g) was added and this solution was reacted at 70°C with stirring for 2 hours. The reaction mixture was treated with Amberlite® XAD—2 produced by Rohm and Hass Co. (700 ml) in a column. By elution with water the fractions containing the desired compound were collected. The solution was lyophilized to give the object compound, 7 - $\beta$ - [D - (—) - $\alpha$ - (4 - carboxyimidazole - 5 - carboxamido) - phenylacetamido] - 3 - (4 - $\beta$ - sulphoethylpyridinium)methyl - 3 - cephem - 4 - carboxylic acid disodium salt trihydrate.
(0.2 g, yield: 14%).

Elemental analysis
Found C, 43.26%, H 3.66%, N 10.61%, S 8.15%, Na 6.0%
Calcd. for $C_{28}H_{24}N_6O_{10}S_2Na_2 \cdot 3H_2O$
C 43.74%, H 3.94%, N 10.93%, S 8.34%, Na 5.98%

I.R. spectrum (Nujol)
$\nu_{C=O}$($\beta$-lactam) = 1770 $cm^{-1}$
$\nu SO_2$(—$SO_3H$) = 1220 $cm^{-1}$, 1045 $cm^{-1}$
N.M.R. spectrum ($D_2O$, $\delta$)

ppm 3.10 (m, 2H) ($CH_2$, 2nd position
3.36 (s, 4H) (—$CH_2CH_2SO_3H$)
5.13 (d, 1H) (—H, 6th position)
5.35 (m, 2H) (—$CH_2$— 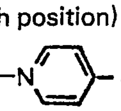, 3rd position)
5.62 (s, 1H) (–CH–)
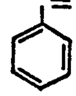

5.77 (d, 1H) (—H, 7th position)

7.50 (m, 5H) ( 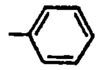 )

7.83 (s, 1H) (—H, 2nd position in imidazole)

7.95 (d, 2H)
8.78 (d, 2H) } ( 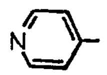 )

Example 9

7$\beta$-[D-(—)-$\alpha$-(4-carboxyimidazole-5-carboxamido)phenylacetamido]-cephalosporanic acid.2Na salt (1.17 g, 2 mM) was reacted with 3-pyridylacetic acid (0.55 g, 4 mM) to give 7$\beta$ - [D - (—) - $\alpha$ - (4 - carboxyimidazole - 5 - carboxamido)phenylacetamido] - 3 - (3 - carboxymethylpyridinium) methyl-3-cephem-4-carboxylic acid disodium salt (0.17 g, yield: 12%) in the same manner as in example 8.

I.R. spectrum (Nujol)
$V_{c=o}$ ($\beta$-lactam) = 1760 $cm^{-1}$
N.M.R. spectrum ($D_2O$, $\delta$)

ppm 3.35 (m, 2H) (>$CH_2$, 2nd position)

3.60 (s, 2H) 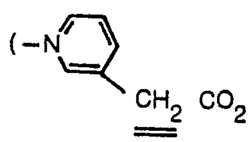

5.10 (d, 1H) (—H, 6th position)

5.60 (s, 1H) 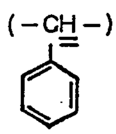

5.73 (d, 1H) (—H, 7th position)

7.45 (m, 5H) ( 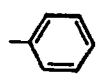 )

7.80 (s, 1H) (—H, 2nd position in imidazole)

7.90 & 8.40 (m, 4H) 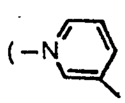 )

Imidazole dicarboxylic acid derivatives obtained by the process of the present invention have a marked antibacterial activity within a wide range, particularly against Pseudomonas aeruginosa.

Comparative MIC's for compounds using Pseudomonas aeruginosa AJ 2116 as the test organism are as follows:

| Example of this Application | MIC (μg/ml) |
|---|---|
| 3 | 50 |
| 4 | 12.5 |
| 5 | 12.5 |
| 6 | 50 |
| 7 | 50 |
| 8 | 12.5 |
| 9 | 12.5 |
| Carbenicillin | 100 |
| Ampicillin | More than 500 |

"MIC" represents the Minimum Inhibitory concentration in mcg./ml of the compound required to inhibit the growth of the test organism described.

**Claims**

1. An imidazoledicarboxylic acid derivative having the following formula:

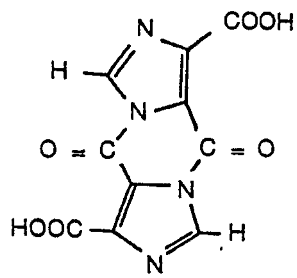

(I)

2. A process for producing a compound having the following formula:

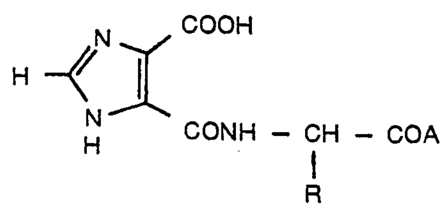

(II)

or a pharmaceutically acceptable salt thereof, wherein A is an organic residue having one of the following formulae:

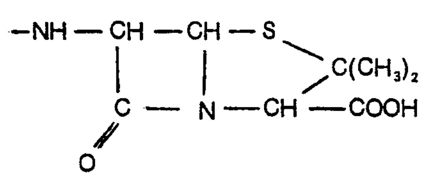

and

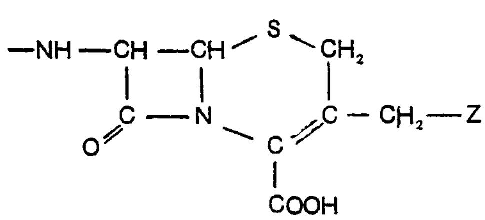

in which the carboxylic groups are possibly esterified, Z is a hydrogen atom, an acyloxy group, a carbamoyloxy group, a heteroaromatic thio group or a quaternary ammonium group, and R is a hydrogen atom, an alkyl group, an aralkyl group, an aryl group or a heteroaromatic group, which may have substituent group or groups, characterized in that an imidazoledicarboxylic acid derivative having the following formula

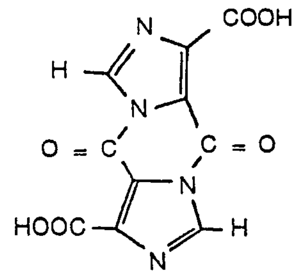

is reacted with a compound having the following formula:

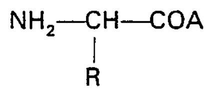

wherein A and R are defined as above and, if present, ester groups are optionally removed by conventional methods and/or a pharmaceutically acceptable salt is optionally formed.

3. A process according to claim 2, wherein R is a group of the formula

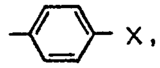

wherein X is a hydrogen atom or a hydroxyl group.

**Revendications**

1. Dérivé de l'acide imidazoledicarboxylique de formule:

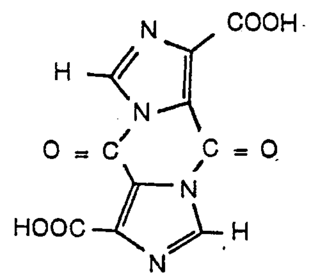

(I)

2. Procédé de synthèse d'un corps de formule suivante:

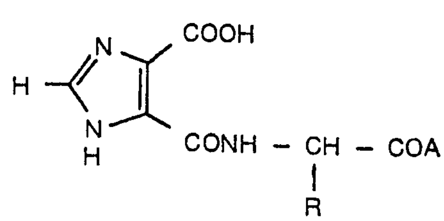

(II)

ou d'un sel de celui-ci qui soit acceptable du point de vue pharmaceutique, dans cette formule A est un résidu organique dont la structure correspond à l'une des formules suivantes:

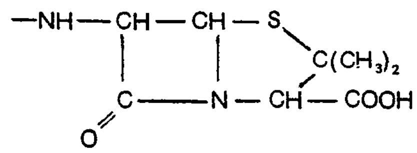

et

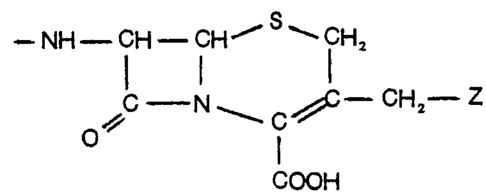

dans lesquelles les groupes carboxyliques peuvent être estérifiés, Z représente un atome d'hydrogène, un groupe acyloxyle, un groupe carbamoyloxyle, un groupe thio hétéroaromatique ou un groupe ammonium quatemaire et R représente un atome d'hydrogène, un groupe alkyle un groupe aralkyle, un groupe aryle ou un groupe hétéroaromatique, qui peuvent posséder un ou plusieurs substituants; ce procédé est caractérisé par le fait que l'on fait réagir un dérivé de l'acide imidazoledicarboxylique de formule:

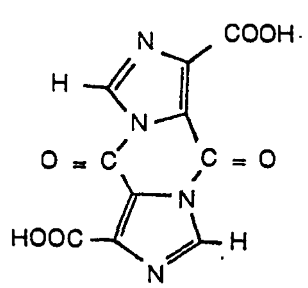

avec un corps de formule:

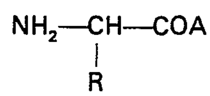

dans laquelle A et R ont la même signification que ci-dessus, et, s'ils sont présents, les groupes esters peuvent être éliminés facultativement par des méthodes conventionnelles et on peut former, si on le désire, un sel qui soit acceptable du point de vue pharmaceutique.

3. Procédé selon la revendication 3, caractérisé en ce que R est un groupe de formule:

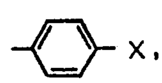

dans laquelle X est un atome d'hydrogène ou un groupe hydroxyle.

**Patentansprüche**

1. Imidazoldicarbonsäurederivat der folgenden Formel

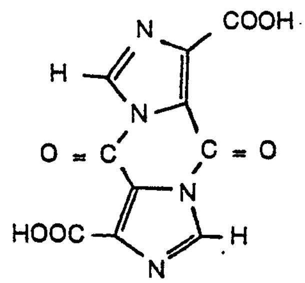

(I)

2. Verfahren zur Herstellung einer Verbindung der allgemeinen Formel

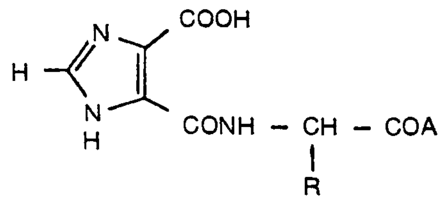

(II)

oder eines pharmazeutisch geeigneten Salzes davon, wobei A ein organischer Rest einer der nachstehenden Formeln ist:

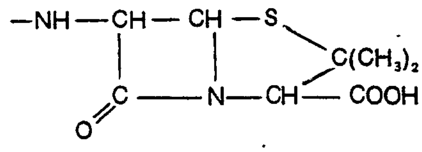

und

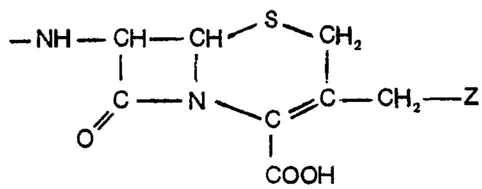

in denen die Carbonsäuregruppen gegebenenfalls verestert sind, Z ein Wasserstoffatom, eine Acyloxygruppe, eine Carbamoyloxygruppe, eine heteroaromatische Thiogruppe oder eine quaternäre Ammoniumgruppe bedeutet und R ein Wasserstoffatom, eine Alkylgruppe, eine Aralkylgruppe, eine Arylgruppe oder eine heteroaromatische Gruppe ist, die eine Substituentengruppe oder Substituentengruppen aufweisen können, dadurch gekennzeichnet, daß ein Imidazoldicarbonsäurederivat der folgenden Formel

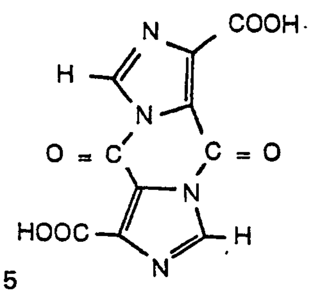

Fig. 4, 5

mit einer Verbindung der folgenden Formel

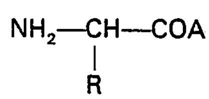

umgesetzt wird, worin A und R die vorstehende Definition haben, und gegebenenfalls Estergruppen, falls solche vorliegen, mit Hilfe üblicher Methoden entfernt werden und/oder gegebenenfalls ein pharmazeutisch geeignetes Salz gebildet wird.

3. Verfahren nach Anspruch 2, dadurch gekennzeichnet, daß R eine Gruppe der Formel

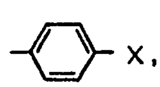

bedeutet, worin X ein Wasserstoffatom oder eine Hydroxylgruppe ist.